# EUROPÄISCHE PATENTSCHRIFT

(11) **EP 3 193 961 B1**
(45) Veröffentlichungstag und Bekanntmachung des Hinweises auf die Patenterteilung: **01.09.2021**
(21) Anmeldenummer: 15766458.2
(22) Anmeldetag: 17.09.2015
(51) Int. Cl.: A61M 1/14, A61M 1/34, G16H 40/63, G16H 20/40, G16H 40/60

(54) **VORRICHTUNG ZUM STEUERN EINER BLUTBEHANDLUNGSVORRICHTUNG UNTER BERÜCKSICHTIGEN VON PATIENTENFEEDBACK BEI DER BLUTBEHANDLUNG UND BLUTBEHANDLUNGSVORRICHTUNG**
DEVICE FOR CONTROLLING A BLOOD TREATMENT DEVICE WHILE TAKING INTO CONSIDERATION PATIENT FEEDBACK DURING THE BLOOD TREATMENT AND BLOOD TREATMENT DEVICE
DISPOSITIF DE COMMANDE D'UN DISPOSITIF DE TRAITEMENT DU SANG EN TENANT COMPTE DES RÉACTIONS DU PATIENT PENDANT LE TRAITEMENT DU SANG, AINSI QUE DISPOSITIF DE TRAITEMENT DU SANG

(30) Priorität: 18.09.2014 DE 102014113462
(43) Veröffentlichungstag der Anmeldung: 26.07.2017
(73) Patentinhaber: Fresenius Medical Care Deutschland GmbH, 61352 Bad Homburg (DE)
(72) Erfinder: DÜLSNER, Erik, 61197 Florstadt (DE); SCHRÖRS, Alexander, 60389 Frankfurt am Main (DE); WEHMEYER, Wolfgang, 72076 Tübingen (DE)
(74) Vertreter: Bobbert & Partner Patentanwälte PartmbB
(86) Internationale Anmeldenummer: PCT/EP2015/071325
(87) Internationale Veröffentlichungsnummer: WO 2016/042078

(56) Entgegenhaltungen:
- EP-A1- 2 433 662
- WO-A1-2010/108955
- DE-A1-102012 020 945
- US-A- 5 401 238
- US-A1- 2009 069 642
- US-A1- 2011 157 480

## Beschreibung

Die vorliegende Erfindung betrifft eine Steuer- und/oder Regelungsvorrichtung gemäß Anspruch 1, ein offenbartes Verfahren sowie eine Blutbehandlungsvorrichtung gemäß Anspruch 12.

Eine Blutbehandlung, in Form beispielsweise einer Dialyse, ist für den Patienten eine oftmals belastende Prozedur. Die Belastung empfindet der Patient u. a. aufgrund körperlicher Symptome wie Abgeschlagenheit, Kopfschmerzen, Übelkeit und weiterer Symptome. Sie alle können sich im Verlauf der Behandlung verstärken, ergänzen oder abwechseln. Die Blutbehandlung als solche kann überdies für den Patienten belastend sein, u. a. aufgrund der Beschränkung, die der Patient durch die Notwendigkeit der Behandlung empfindet, die von ihm zur Behandlung aufzubringende Zeit und der gefühlten Machtlosigkeit gegenüber seiner die Behandlung erforderlich machenden Grunderkrankung. Das Auftreten mancher oder aller der vorgenannten Symptome und ihre Heftigkeit können durch geschickte Steuerung oder Regelung der zur Behandlung verwendeten Blutbehandlungsvorrichtung verhindert oder zumindest abgemildert werden.

Aus der WO 2010/108955 A1 ist eine Dialysevorrichtung bekannt.

In der US 5,401,238 A wird ein Verfahren zum Überwachen einer Dialyseeinheit offenbart.

Eine Vorrichtung, ein System und ein Verfahren zur Überwachung, Informationsanzeige und Bedienung von medizinischen Fluidmanagementgeräten sind aus der DE 20 2012 020945 A1 bekannt.

Aus der EP 2 433 662 A1 ist eine Vorrichtung zur extrakorporalen Behandlung von Blut bekannt.

In der US 2011/0157480 A1 ist ein Integrationssystem für medizinische Instrumente mit Fernsteuerung offenbart.

Ein tragbares drahtloses elektronisches Gerät zur elektronischen Patientendatenkommunikation und physiologischen Überwachung ist aus der US 2009/0069642 A1 bekannt.

Eine Aufgabe der vorliegenden Erfindung ist es, eine weitere Steuer- und/oder Regelungsvorrichtung (im Folgenden auch kurz: Steuervorrichtung) für eine Blutbehandlungsvorrichtung anzugeben.

Ferner soll eine Blutbehandlungsvorrichtung mit einer erfindungsgemäßen Steuervorrichtung angegeben werden.

Die erfindungsgemäße Aufgabe wird durch eine Steuer- und/oder Regelungsvorrichtung mit den Merkmalen des Anspruchs 1 gelöst. Zudem wird sie gelöst durch eine Blutbehandlungsvorrichtung mit den Merkmalen des Anspruchs 12.

Die erfindungsgemäße Steuer- und/oder Regelungsvorrichtung, kurz: Steuervorrichtung, ist mit einer ersten, insbesondere von einem Arzt betätigbaren Eingabevorrichtung in Signalverbindung verbunden oder zu einer Verbindung hiermit vorbereitet. Sie ist ferner mit einer zweiten, insbesondere von einem Patienten betätigbaren Eingabevorrichtung in Signalverbindung verbunden oder zu einer solchen Verbindung vorbereitet. Die erfindungsgemäße Steuervorrichtung ist programmiert, um in Signalverbindung mit einer Blutbehandlungsvorrichtung zu stehen und/oder diese zu einer Blutbehandlung zu veranlassen. Die Blutbehandlungsvorrichtung ist hierzu mittels eines extrakorporalen Blutkreislaufs mit einem Blutfilter verbunden.

Die erfindungsgemäße Steuervorrichtung ist weiter programmiert, um die Blutbehandlung des Patienten - oder die Blutbehandlungsvorrichtung - unter Ausführen wenigstens der im Folgenden genannten Schritte zu steuern und/oder zu regeln (im Folgenden auch vereinfachend kurz als "steuern" bezeichnet).

So wird die Blutbehandlungsvorrichtung zur Blutbehandlung des Patienten mit einer ersten, hier als Ausgangseinstellung bezeichneten Einstellung für wenigstens einen Behandlungsparameter gesteuert. Die erste Einstellung ist jene mittels der ersten Eingabevorrichtung eingestellte, eingegebene oder voreingestellte Eingabe oder Anweisung an die Blutbehandlungsvorrichtung.

Die Steuervorrichtung ist programmiert, um einmalig, mehrmalig, regelmäßig, insbesondere in vorbestimmten Zeitabständen zu überprüfen, ob mittels der zweiten Eingabevorrichtung eine Eingabe erfolgt ist.

Wird dabei festgestellt, dass eine solche Eingabe erfolgt ist, so wertet die Steuervorrichtung aufgrund ihrer Programmierung die erfolgte Eingabe aus; ist keine Eingabe erfolgt, unterbleibt die Auswertung. Alternativ oder ergänzend zu dieser Auswertung wird von der Steuervorrichtung eine zweite, hier als Zieleinstellung bezeichnete Einstellung für den wenigstens einen Behandlungsparameter festgelegt. Das Festlegen basiert auf - oder erfolgt in Abhängigkeit von - der mittels der zweiten Eingabevorrichtung erfolgten Eingabe (falls eine solche Eingabe erfolgt ist, sonst ist dieser Schritt nicht erforderlich); diese Eingabe wird beim Festlegen berücksichtigt, beispielsweise auf rechnerische Weise oder durch Rückgriff auf eine Nachschlagetabelle.

Wurde mittels der Steuervorrichtung eine Zieleinstellung festgelegt, so steuert die Steuereinrichtung die Blutbehandlungsvorrichtung - vorzugsweise ab dem Zeitpunkt des Festlegens, ggf. aber auch zeitverzögert - derart, dass die weitere Blutbehandlung des Patienten mit der Zieleinstellung, also mit der zweiten Einstellung, erfolgt.

Das Festlegen der Zieleinstellung kann als ein Anpassen, Ändern oder Überschreiben der Ausgangseinstellung verstanden werden.

Das offenbarte Verfahren zum Steuern einer Blutbehandlungsvorrichtung umfasst ein Bereitstellen einer, vorzugsweise erfindungsgemäßen, Steuer- und/oder Regelungsvorrichtung, welche mit einer ersten, insbesondere von einem Arzt betätigbaren Eingabevorrichtung und mit einer zweiten, insbesondere von einem Patienten betätigbaren Eingabevorrichtung jeweils in Signalverbindung verbunden ist.

Die Steuer- oder Regelungsvorrichtung ist programmiert, um in Signalverbindung mit einer Blutbehandlungsvorrichtung, welche wiederum mit einem Blutfilter verbunden ist, eine Blutbehandlung des Patienten unter Ausführen folgender Schritte zu steuern und/oder zu regeln:
- Steuern und/oder Regeln der Blutbehandlungsvorrichtung zur Blutbehandlung des Patienten mit einer ersten, als Ausgangseinstellung bezeichneten, mittels der ersten Eingabevorrichtung eingestellten Einstellung für wenigstens einen Behandlungsparameter;
- Überprüfen, ob mittels der zweiten Eingabevorrichtung eine Eingabe erfolgt ist;
- Auswerten der mittels der zweiten Eingabevorrichtung erfolgten Eingabe und/oder Festlegen einer zweiten, als Zieleinstellung bezeichneten Einstellung für den wenigstens einen Behandlungsparameter basierend auf der mittels der zweiten Eingabevorrichtung erfolgten Eingabe; und
- Steuern oder Regeln der Blutbehandlungsvorrichtung zur Blutbehandlung des Patienten mit der zweiten Einstellung und/oder Steuern oder Regeln der Blutbehandlungsvorrichtung unter Anpassen oder Ändern der Ausgangseinstellung entsprechend der Eingabe oder der Zieleinstellung.

Die erfindungsgemäße Blutbehandlungsvorrichtung weist einen extrakorporalen Blutkreislauf und/oder eine Blutkassette auf, oder ist hiermit verbunden. Die Blutbehandlungsvorrichtung weist weiter wenigstens eine erfindungsgemäße Steuer- und/oder Regelungsvorrichtung auf.

Erfindungsgemäße Ausführungsformen können manche, einige oder alle der folgenden Merkmale in beliebiger Kombination aufweisen, soweit dies für den Fachmann nicht erkennbar technisch unmöglich ist. Zu einer erfindungsgemäßen Vorrichtung hierin genannte Merkmale können mit dem Verfahren kombiniert werden. Vorteilhafte Weiterbildungen der vorliegenden Erfindung sind jeweils auch Gegenstand der Unteransprüche.

Bei allen oben gemachten und allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin die Rede von "geeignet", "vorgesehen", "ausgelegt", "konfiguriert" und/oder "programmiert" ist, kann der Fachmann dies als eine besondere Ausgestaltung der betreffenden Vorrichtung verstehen. Die vorgenannten Begriffe können hier austauschbar verwendet werden.

Obgleich die Erfindung im Folgenden vornehmlich anhand der Dialysebehandlung beschrieben wird, ist die Erfindung hierauf nicht beschränkt.

In einigen erfindungsgemäßen, beispielhaften Ausführungsformen ist die Steuervorrichtung programmiert, um eine Eingabe mittels der zweiten Eingabevorrichtung, das Festlegen der Zieleinstellung und/oder das Anpassen oder Ändern der Ausgangseinstellung des wenigstens einen Behandlungsparameters nur innerhalb eines zuvor - beispielsweise werkseitig oder vom Personal und/oder mittels der ersten Eingabevorrichtung - festgelegten Rahmens oder Bereichs oder innerhalb zuvor festgelegter Grenzen zuzulassen.

In gewissen erfindungsgemäßen, beispielhaften Ausführungsformen ist die Steuervorrichtung programmiert, um einen Rahmen oder Bereich oder Grenzen, innerhalb derer das Eingeben mittels der zweiten Eingabevorrichtung, das Festlegen und/oder das Anpassen des wenigstens einen Behandlungsparameters ermöglicht wird, oder ist, eingeben zu können.

In bestimmten erfindungsgemäßen, beispielhaften Ausführungsformen ist der Behandlungsparameter ausgewählt aus der Gruppe, bestehend aus: Fluss der Dialysierflüssigkeit, Blutfluss durch den Blutfilter; Zufluss oder -rate einer Infusionslösung, Bereitstellen oder Konzentration von osmostisch wirksamen Substanzen in der Dialysierflüssigkeit oder dem Substituat, Konzentration von Bikarbonat, Natrium, Kalium, Magnesium und/oder Kalzium jeweils in der Dialysierflüssigkeit oder dem Substituat, Filtrationsrate, Ultrafiltrationsrate, Zeitdauer der Blutbehandlung, Temperatur des dem Patienten rückinfundierten Bluts und/oder Temperatur der Dialysierflüssigkeit. Die Temperatur ist beispielsweise jene einer Quelle für Dialysierflüssigkeit oder jene der Dialysierflüssigkeit bei dessen Eintritt in den Blutfilter oder in den extrakorporalen Blutkreislauf.

Die Blutbehandlungsvorrichtung kann erfindungsgemäß alle zum Durchführen der Steuerung und/oder Regelung erforderlichen Vorrichtungen wie Blutpumpe, Ultrafiltrationspumpe oder dergleichen aufweisen. Die Steuervorrichtung kann mit diesen Vorrichtungen jeweils in Signalverbindung stehen.

Zu den vom Patienten in gewissen erfindungsgemäßen Ausführungsformen beeinflussbaren Behandlungsparametern zählt somit - direkt oder indirekt - auch die Temperatur, mit der das Blut dem Patienten zurückgegeben wird. Sie ist zum einen ein Faktor, welcher das Wohlbefinden des Patienten beeinflusst. Zum anderen hat Bluttemperatur aber auch einen therapeutischen Effekt, den der Patient mittels seiner Eingaben ebenfalls beeinflussen kann.

Der Arzt eines Dialysepatienten tendiert beispielsweise, wenn er die Temperatur, die die Dialysierflüssigkeit haben soll, festlegt, i. d. R. dazu, sie so festzulegen, dass dies zu einer leichten Kühlung oder Unterkühlung des Patienten führt. Dies geschieht in Absicht, um zum einen die durch die Ultrafiltration im Körperkern entstehende Wärme abzuführen, zum anderen, um durch eine leichte, durch die Kühle bedingte Vasokonstriktion der peripheren Gefäße den Blutdruck bei der Dialyse zu stützen. Liegt die Temperatur zu tief, beklagt sich der Patient über Frieren. Des Weiteren muss in diesem Fall mit einer gewissen Einschränkung der Dialysequalität gerechnet werden, da durch die Vasokonstriktion ein Pool des Körpers nicht gut entgiftet wird. Liegt die Temperatur zu hoch, kann es während der Dialyse aufgrund von Vasodilatation zu einem bedrohlichen Abfall des Blutdrucks kommen.

Für die vorliegende Erfindung wird vorzugsweise vorgeschlagen, dass der Arzt zunächst und individuell für jeden Patienten einen Temperaturbereich vorgibt, der die zuvor genannten Effekte vermeidet. Dieser Temperaturbereich ist kleiner als der an der Blutbehandlungsvorrichtung einstellbare Temperaturbereich. Die Dialyse wird dann mit einem Temperaturwert für die Dialysierflüssigkeit gestartet, der z. B. den Mittelwert dieser Grenzen darstellt.

Der Patient kann mittels der zweiten Eingabevorrichtung die Temperatur der Dialysierflüssigkeit während der Behandlung - und vorzugsweise innerhalb der vom Arzt vorgegebenen Grenzen - beliebig manipulieren.

Dabei ist es in bestimmten erfindungsgemäßen, beispielhaften Ausführungsformen nicht erforderlich, dass der Patient beim Verändern der Temperatur die absolute Temperatur kennt. Diese muss hierzu folglich auch nicht bestimmt oder ihm mitgeteilt werden. Es kann ausreichend sein, wenn der mittels der zweiten Eingabevorrichtung veränderbare Parameter relativ ausgedrückt wird, etwa als "wärmer" oder "kälter".

In einigen erfindungsgemäßen, beispielhaften Ausführungsformen wird eine Zieleinstellung nur temporär zur Grundlage der Steuerung verwendet. Durch schrittweise oder kontinuierliche Anpassung wird Steuerung nach einer Eingabe des Patienten mittels der zweiten Eingabevorrichtung wieder basierend auf der Ausgangseinstellung betrieben. Die Zeitdauer, ab welcher dies der Falls sein soll, kann einstellbar sein. Der Übergang von Zieleinstellung auf Ausgangseinstellung kann schrittweise nach zuvor festgelegten Muster erfolgen, er kann stufenartig erfolgen, usw.

In manchen erfindungsgemäßen, beispielhaften Ausführungsformen werden der Rahmen oder Bereich oder die Grenzen, innerhalb derer das Eingeben mittels der zweiten Eingabevorrichtung, das Festlegen und/oder das Anpassen des wenigstens einen Behandlungsparameters ermöglicht wird, - beispielsweise ergänzend oder alternativ - in Abhängigkeit vom Ergebnis von während der Blutbehandlung erfolgender Blutdruckmessungen automatisch weiter verändert, vorzugsweise weiter eingeschränkt. Hierzu kann das Ergebnis der Blutdruckmessung mit für diesen Patienten zuvor als zulässig festgelegten Blutdruckgrenzwerten verglichen werden. Werden die Grenzen unter- oder überschritten, wird der für eine Eingabe oder Zieleinstellung verfügbare Bereich des Parameters, etwa der Temperatur der Dialysierflüssigkeit, angepasst.

In gewissen erfindungsgemäßen, beispielhaften Ausführungsformen basiert die zweite Eingabevorrichtung auf einer mechanischen und/oder elektrischen Lösung. Sie kann z. B. ein Linear- oder Drehpotentiometer sein. Sie kann ein Schalter oder Kippschalter sein. Sie kann eine akustische Eingabevorrichtung (Spracheingabe) sein. Sie kann mittels einer Fernsteuerung und/oder Gestensteuerung bedienbar sein. Sie kann einen Bildschirm aufweisen und/oder mittels Berührung (z. B. als touch-panel) bedienbar sein.

In manchen erfindungsgemäßen, beispielhaften Ausführungsformen ist die zweite Eingabevorrichtung ausgestaltet oder konfiguriert, um - wenigstens oder ausschließlich - eine qualitative Eingabe zu erlauben, welche eine qualitative Anpassung bewirkt oder hierfür codiert. Die Steuervorrichtung ist dabei programmiert, um die mittels der zweiten Eingabevorrichtung erfolgte, qualitative Eingabe nach vorbestimmten Routinen oder Regeln in quantitative Zieleinstellungen zu transformieren.

In gewissen erfindungsgemäßen, beispielhaften Ausführungsformen erlaubt die zweite Eingabevorrichtung keine quantitative Einstellung.

Qualitative Eingaben können beispielsweise als "wärmer" oder "kälter" bezeichnet sein, siehe oben. Sie können aber auch als "schonender" oder "effizienter", "langsamer" oder "schneller", oder dergleichen bezeichnet sein.

Jede quantitative Eingabe kann gemäß hinterlegter Regeln oder Routinen zu für die jeweilige Eingabe vorhersagbaren Änderungen der Steuerung hinsichtlich eines oder mehrerer einstellbarer Behandlungsparameter führen. So kann die Betätigung eines "schonender"-Schalters wie vorab festgelegt zu einer Senkung der Pumpleistung der Ultrafiltrationsleistung (und ggf. auch noch weiterer Behandlungsparameter) führen, während die Betätigung eines "kälter"-Schalters zu einer Senkung der Temperatur der Dialysierflüssigkeit führen kann.

Die zweite Eingabevorrichtung bietet dem Patienten in gewissen, erfindungsgemäßen, beispielhaften Ausführungsformen mehr als nur eine, ggf. mit entgegengesetzter Wirkung, hinterlegte qualitative Eingabemöglichkeit. Vielmehr kann die Eingabevorrichtung ausgestaltet sein, um neben einer "wärmer/kälter"-Eingabe zusätzlich auch eine "schonender/effizienter"-Eingabe zu erlauben, usw.

In einigen erfindungsgemäßen, beispielhaften Ausführungsformen ist die Steuervorrichtung derart ausgestaltet, dass man bei der Eingabe mittels der zweiten Eingabevorrichtung zwischen wenigstens oder ausschließlich einer ersten und einer zweiten Eingabeoption auswählen kann, welche vorzugsweise jeweils ein zueinander entgegengesetztes Anpassen oder Ändern der Ausgangseinstellung des Behandlungsparameters bewirken, etwa gemessen an der aktuellen Ausprägung oder Ausgangseinstellung des Behandlungsparameters oder Blutbehandlungsvorrichtung.

In manchen erfindungsgemäßen, beispielhaften Ausführungsformen bewirkt das Anpassen oder Ändern der Ausgangseinstellung eine vorbestimmte, insbesondere werte- oder zahlenmäßig feste Änderung des Behandlungsparameters.

In bestimmten erfindungsgemäßen, beispielhaften Ausführungsformen ist das Anpassen oder Ändern der Ausgangseinstellung eine variable Anpassung, etwa mittels Regler, Touchscreen, Tableteingabe oder Schieber, bei welcher vorzugsweise ein stufenloses, möglichst jedoch vorzugsweise ein zumindest mehrstufiges Anpassen möglich ist (beispielsweise, indem für die Anpassung mindestens 3, 4 oder mehr auswählbare Stufen vorgesehen sind).

In manchen erfindungsgemäßen, beispielhaften Ausführungsformen ist die Steuervorrichtung programmiert, um das Anpassen oder Ändern der Ausgangseinstellung des Behandlungsparameters und/oder das Festlegen der Zieleinstellung in Abhängigkeit von der seit Beginn der Blutbehandlung verstrichenen Zeit zu bewirken.

So kann beispielsweise vorgesehen sein, dass die Betätigung eines "effektiver"-Schalters als zweite Eingabevorrichtung durch den Patienten zu einem früheren Zeitpunkt der Behandlungssitzung eine sanftere Erhöhung beispielsweise der Pumpraten zur Folge hat, als dieselbe Eingabe zu einem späteren Zeitpunkt. Diese Ausgestaltung basiert beispielsweise auf dem Gedanken, dass das sanftere Erhöhen der Pumpraten zum früheren Zeitpunkt dieselbe Wirkung haben kann wie ein stärkeres Erhöhen der Pumpraten zu einem späteren Zeitpunkt, zu dem bei festgelegtem Endzeitpunkt der Behandlung zum Erzielen der höheren Effizienz eine vergleichsweise kürze Zeitdauer verbleibt.

In einigen erfindungsgemäßen, beispielhaften Ausführungsformen ist die Steuervorrichtung programmiert, um ein mittels der zweiten Eingabevorrichtung zu einem ersten Zeitpunkt oder in einem ersten Zeitraum während der Blutbehandlung erfolgtes Anpassen oder Ändern der Ausgangseinstellung des Behandlungsparameters zu einem zweiten Zeitpunkt oder in einem zweiten Zeitraum während der Blutbehandlung zu kompensieren. Hierzu ist die Steuervorrichtung programmiert, um die Blutbehandlung zum zweiten Zeitpunkt oder im zweiten Zeitraum automatisch mit einer dritten, hier als Korrektureinstellung bezeichneten Einstellung zu steuern oder zu regeln. Der zweite Zeitpunkt oder Zeitraum liegt hierbei nach dem ersten Zeitpunkt bzw. Zeitraum.

In manchen erfindungsgemäßen, beispielhaften Ausführungsformen wird die Korrektureinstellung derart bestimmt, dass ein mittels Ausgangseinstellung angestrebtes Ziel der Blutbehandlung, insbesondere während einer vorbestimmten Zeitdauer der Blutbehandlung, welche trotz Eingabe durch den Patienten, erreicht wird.

In gewissen erfindungsgemäßen, beispielhaften Ausführungsformen wird die Korrektureinstellung derart bestimmt, dass ein mittels Ausgangseinstellung angestrebtes Ziel der Blutbehandlung innerhalb einer ursprünglich, beispielsweise vor oder zu Beginn der Blutbehandlung, festgesetzten Behandlungsdauer erreicht wird.

In gewissen erfindungsgemäßen, beispielhaften Ausführungsformen ist das angestrebte, vorzugsweise vorbestimmte oder vordefinierte, Ziel ein Hydrierungszustand und/oder ein Entgiftungszustand, welcher wiederum vorbestimmt oder vordefiniert sein kann.

Der Hydrierungszustand des Patienten gibt bekanntermaßen die Belastung des Patienten durch Wasser an, welches im Fall der Überwässerung nicht über die Nieren abgeführt wurde und welches im Fall der Dehydrierung dem Körper - gemessen an einer physiologischen Hydrierung - fehlt. Hyperhydrierung (oder Überwässerung) und Dehydrierung (oder Wassermangel) sind beides Zeichen für eine abnormale Flüssigkeitsvolumenregelung, wie dies beispielsweise in Guyton AC, Hall JE, Physiology, Elsevier & Saunders, 11ed, (2006), Seite 301 erläutert ist. Verfahren und Vorrichtung zum Messen des Hydrierungszustandes sind beispielsweise aus der WO 2006/002685 A1 bekannt. Eine Überwässerung wird darin beispielsweise als *a*ECW* + *b*ICW* + *c*body weight* berechnet mit *ECW* als extrazellulärem Wasser, *ICW* als intrazellulärem Wasser, *a, b* und c als Variablen oder Konstanten und *body weight* als Körpergewicht.

Der Entgiftungszustand ist jener Zustand, zu welchem eine physiologische oder vor Beginn der Blutbehandlung als Behandlungsziel festgelegte Konzentration einer oder mehrerer Substanzen des Bluts, wie Harnstoff, Kalium oder Natrium im Blut erreicht ist.

In bestimmten erfindungsgemäßen, beispielhaften Ausführungsformen wird die Korrektureinstellung derart bestimmt, dass eine Auswirkung des Abweichens von der Ausgangseinstellung, bewirkt durch das Steuern entsprechend der Zieleinstellung über eine gewisse Zeit, zumindest teilweise kompensiert, aufgehoben wird oder der Auswirkung des Abweichens entgegengewirkt wird.

In manchen erfindungsgemäßen, beispielhaften Ausführungsformen ist die Steuervorrichtung programmiert, um nach erfolgtem Steuern oder Regeln der Blutbehandlungsvorrichtung mit der zweiten Einstellung oder der Zieleinstellung automatisch zumindest teilweise von der Zieleinstellung zur Ausgangseinstellung zurückzukehren. Dies kann beispielsweise progressiv erfolgen. Es kann vollständig oder nur teilweise erfolgen.

In bestimmten erfindungsgemäßen, beispielhaften Ausführungsformen ist die Steuervorrichtung programmiert, um nach begonnenem Steuern oder Regeln der Blutbehandlungsvorrichtung mit der zweiten Einstellung den voreingestellten oder von der Steuer- oder Regelungsvorrichtung errechneten Endzeitpunkt in Abhängigkeit von der Zieleinstellung nach hinten zu verschieben. Alternativ kann die voreingestellte oder von der Steuer- oder Regelungsvorrichtung errechnete Behandlungsdauer in Abhängigkeit von der Zieleinstellung automatisch verlängert werden.

In manchen erfindungsgemäßen, beispielhaften Ausführungsformen ist die Steuervorrichtung derart programmiert, dass eine mittels der zweiten Eingabevorrichtung erfolgte Eingabe und/oder das Festlegen der Zieleinstellung nur erfolgt, wenn die Eingabe während wenigstens einer vorbestimmten Zeitphase der Blutbehandlung erfolgt. In anderen Zeitphasen als der oder den vorbestimmten ist eine solche Eingabe nicht möglich, wird nicht ausgewertet oder führt nicht zum Festlegen einer Zieleinstellung.

In gewissen erfindungsgemäßen, beispielhaften Ausführungsformen ist die Steuervorrichtung derart programmiert, dass eine mittels der zweiten Eingabevorrichtung erfolgte Eingabe mittels Sprache und/oder Geste erfolgen kann.

In einigen erfindungsgemäßen, beispielhaften Ausführungsformen weist die Steuervorrichtung eine Ausgabevorrichtung zum Ausgeben und/oder Anzeigen eines optischen oder akustischen Feedbacks oder Darstellung dieser Eingabe bzw. ihrer Konsequenzen (z. B. Änderungen des Kt/V oder der Ultrafiltrationsrate) nach ihrer Berechnung zur Kenntnisnahme durch Patient oder Pflegepersonal auf. Mitgeteilt werden können dabei auch weitere Aspekte der Eingabe, wie etwa eine aufgrund der Eingabe erforderlich gewordene Verlängerung der Behandlungsdauer oder andere Auswirkungen der Eingabe auf die Behandlung. Dies stellt den Patienten vorteilhaft frühzeitig auf den weiteren, beispielsweise gegenüber der Ausgangsplanung geänderten Behandlungsverlauf ein.

In bestimmten erfindungsgemäßen, beispielhaften Ausführungsformen weist die Steuervorrichtung wenigstens eine erste Eingabevorrichtung und eine zweite Eingabevorrichtung auf oder ist mit beiden verbunden, welche jeweils konfiguriert sind zum Einstellen oder Verändern von Einstellungen wenigstens eines - insbesondere ein- und desselben - Behandlungsparameters.

In bestimmten erfindungsgemäßen, beispielhaften Ausführungsformen ist die Blutbehandlungsvorrichtung eine Hämodialysevorrichtung, Hämofiltrationsvorrichtung oder Hämodiafiltrationsvorrichtung.

Die erfindungsgemäße Steuervorrichtung ist in einigen erfindungsgemäßen Ausführungsformen als Regelvorrichtung ausgestaltet.

Die erfindungsgemäße Blutbehandlungsvorrichtung weist in bestimmten erfindungsgemäßen Ausführungsformen wenigstens eine erfindungsgemäße Steuervorrichtung auf.

Manche oder alle erfindungsgemäßen Ausführungsformen können einen, mehrere oder alle der oben und/oder im Folgenden genannten Vorteile aufweisen.

Alle mit der erfindungsgemäßen Steuervorrichtung erzielbaren Vorteile lassen sich in bestimmten erfindungsgemäßen Ausführungsformen ungeschmälert auch mit der erfindungsgemäßen Blutbehandlungsvorrichtung erzielen.

Ein mittels mancher Ausführungsformen der vorliegenden Erfindung erzielbarer Vorteil besteht darin, dass der Patient verstärkt in die Kontrolle seiner Blutbehandlung, etwa einer Dialysebehandlung, eingebunden werden kann. Er erhält dadurch die Möglichkeit, mehr Verantwortung für sich selbst zu übernehmen und durch sein Mitwirken eine positivere Einstellung zur oftmals belastenden Behandlungssituation zu gewinnen.

Durch die Einflussnahme kann sich der Patient emotional ein wenig aus seiner regelmäßig als hilflos empfundenen Situation lösen und sich selbst wieder als handelnde Person erleben, wodurch er zu einem kompetenteren Gesprächspartner für den Arzt und das Pflegepersonal wird.

Zudem kann der Patient bei Nutzung der zweiten Eingabevorrichtung der erfindungsgemäßen Vorrichtungen dem Personal Arbeit abnehmen, indem er von ihm für erforderlich gehaltene Anpassungen seiner Behandlung in gewissem Maße selber vornehmen kann. Die vorliegende Erfindung ermöglicht daher vorteilhaft eine zeitliche Entlastung des Personals, ein verbessertes Wohlempfinden des Patienten durch vom ihm durchführbare, genauere und häufigere Anpassung der Blutbehandlungseinstellungen, bei zugleich höherer, i. d. R. als angenehm empfundener Stärkung der Eigenverantwortung des Patienten.

Bekanntermaßen fühlt sich jeder Mensch, jeden Tag, je nach Verfassung verschieden. Die immer gleichen Einstellungen der Blutbehandlungsparameter an der Blutbehandlungsvorrichtung nehmen darauf aber nur bedingt Rücksicht. Patienten können Blutbehandlungsparameter erfindungsgemäß vorteilhaft nun aber so anpassen, dass sie ihrer Tagesform entsprechend behandelt werden.

Ein vorteilhafter Aspekt der vorliegenden Erfindung ist es, dem Blutbehandlungspatienten, beispielsweise dem Dialysepatienten, eine Möglichkeit zur Beeinflussung seiner Behandlung zu bieten. Die Möglichkeit der Beeinflussung kann erfindungsgemäß auf einen Rahmen innerhalb von therapeutischen Grenzen begrenzt werden. Dabei nimmt man in manchen erfindungsgemäßen Ausführungsformen in Kauf, das vormals angestrebte Behandlungsziel (im Fall der Dialyse etwa die Dialysedosis oder die Entwässerung) eventuell nicht zu erreichen.

Durch die Erfindung kann der Patient mittels der zweiten Eingabevorrichtung die Blutbehandlung, etwa die Dialysebehandlung, aber auch derart vorteilhaft beeinflussen, dass die vom Arzt mittels der ersten Eingabevorrichtung vorgegebenen Therapieziele übererfüllt werden. Dies ist beispielsweise dann der Fall, wenn der Patient besonders kreislaufstabil oder auf andere Weise "robust" auf hocheffektive Behandlungen reagiert. Diese "Robustheit" wird vom Patienten anhand seines aktuellen Wohlbefindens selbst festgestellt, dennoch bleibt der Patient unter ständiger Kontrolle durch Überwachung von physiologischen Parametern, wie beispielsweise dem Blutdruck.

Der Patient kann weiter rechtzeitig, u. U. lange bevor es zum Zusammenbruch des Kreislaufes mit teils schwerwiegenden Konsequenzen kommt, direkt auf die Belastung durch die Blutbehandlung Einfluss nehmen. Dadurch werden Komplikationen vermieden, wie z. B. Notfallbolus zur Blutdruckerhöhung (Zielgewicht wird hierdurch bei Dialysepatienten nachteilig i. d. R. nicht erreicht), oder Bildung von Stenosen als Folge des Blutdruckabfalls (verkürzte Lebenszeit des Gefäßzugangs).

Bei Auswertung des Zeitpunktes, an dem der Patient mittels Betätigen der zweiten Eingabevorrichtung eingreift, kann die erfolgversprechendste Gegenmaßnahme zur Verringerung der Belastung durch die Behandlung automatisch eingeleitet werden. Die Automatisierung kann das Training des Pflegepersonals für die sichere Durchführung der angezeigten Erstmaßnahme unterstützen. Es kann das Pflegepersonal ferner weiter entlasten.

Die Eingabe von Einstellungen für die Blutbehandlung an der Blutbehandlungsvorrichtung kann vorteilhaft in der Sprache der Symptomatik des Patienten erfolgen. Es bedarf in solchen Ausführungsformen nicht der Eingabe oder Veränderung von Einstellungen technischer Parameter. Das Wissen über die Umsetzung von Symptomatik in technische Parameter muss nicht mehr trainiert werden, sondern wird vorteilhaft von der erfindungsgemäßen Steuervorrichtung zur Verfügung gestellt.

Während einer Dialyse muss die Temperatur der Dialysierflüssigkeit so eingestellt werden, dass dem Patienten möglichst weder Wärme zugeführt, noch wesentlich Wärme entzogen wird. Zu diesem Zweck wird die Dialysierflüssigkeitstemperatur vom Arzt für die Dialyse vorgegeben.

Je nach Umgebungsbedingungen (z. B. an einem heißen Sommertag oder einem kalten Wintertag) kann eine vorgenommene Einstellung zum Frieren oder Schwitzen des Patienten führen. Eine Anpassung der Temperatureinstellung kann daher während der Dialyse erforderlich sein. Die Einstellung kann bislang technisch und organisatorisch nur von der Schwester ausgeführt werden.

Erfindungsgemäß kann nun aber auch der Patient, mittels der zweiten Eingabevorrichtung, die Temperatur zu jedem Zeitpunkt seinem Wohlempfinden anpassen, ohne dass es zu einem Konflikt mit der Intention der ärztlichen Verschreibung kommt.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße Blutbehandlungsvorrichtung mit einem extrakorporalen Blutkreislauf in einer ersten Ausführungsform;
- **Fig. 2**: zeigt in vereinfachter Darstellung einen exemplarischen, mittels der erfindungsgemäßen Blutbehandlungsvorrichtung erfolgenden Behandlungsverlauf in einer ersten Ausführungsform;
- **Fig. 3**: zeigt in vereinfachter Darstellung einen exemplarischen, mittels der erfindungsgemäßen Blutbehandlungsvorrichtung erfolgenden Behandlungsverlauf in einer zweiten Ausführungsform; und
- **Fig. 4**: zeigt in vereinfachter Darstellung einen exemplarischen, mittels der erfindungsgemäßen Blutbehandlungsvorrichtung erfolgenden Behandlungsverlauf in einer dritten Ausführungsform.

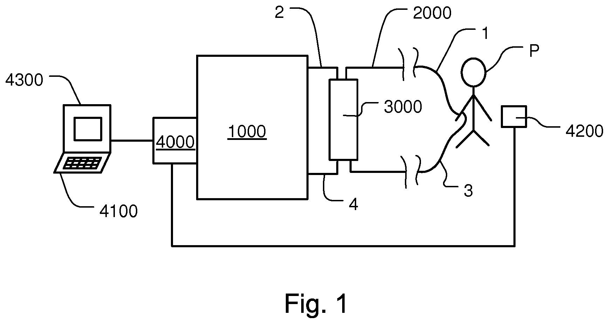

**Fig. 1** zeigt in vereinfachter Darstellung eine erfindungsgemäße Blutbehandlungsvorrichtung 1000 mit einer nicht gezeigten Quelle für Dialysierflüssigkeit und einem extrakorporalen Blutkreislauf 2000.

Der extrakorporale Blutkreislauf 2000 weist eine Blutbehandlungseinrichtung 3000, hier exemplarisch ein Blutfilter oder Dialysator, auf oder ist mit dieser verbunden. Ein arterieller Leitungsabschnitt 1 des extrakorporalen Blutkreislaufs 2000 führt Blut vom Gefäßsystem des Patienten P in Richtung zum Blutfilter 3000. Ein venöser Leitungsabschnitt 3 des extrakorporalen Blutkreislaufs 2000 führt Blut vom Blutfilter 3000 in Richtung zum Gefäßsystem des Patienten P.

Die Blutbehandlungsvorrichtung 1000 weist eine Steuer- und/oder Regelvorrichtung 4000 (kurz: Steuervorrichtung 4000) zum Steuern und/oder Regeln der mittels der Blutbehandlungsvorrichtung 1000 ablaufenden Blutbehandlung auf oder ist mit dieser in Signalverbindung verbunden.

Die Steuervorrichtung 4000 steht wiederum mit einer ersten Eingabevorrichtung 4100 und einer zweiten Eingabevorrichtung 4200 in Signalverbindung. Eine Anzeigevorrichtung 4300, auf welcher Einstellungen zu Behandlungsparametern, der Verlauf der Blutbehandlung oder mittels der ersten und/oder der zweiten Eingabevorrichtungen 4100, 4200 gemachten Eingaben dargestellt werden können, kann vorgesehen sein.

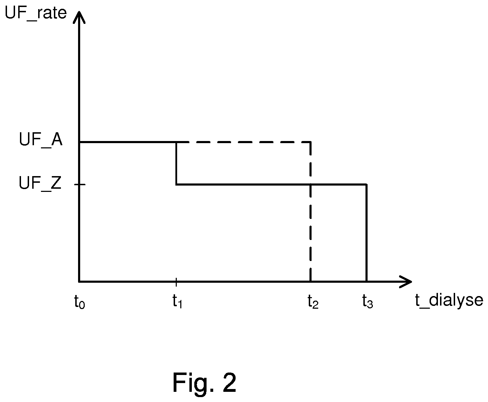

**Fig. 2** zeigt in vereinfachter Darstellung einen exemplarischen, mittels der erfindungsgemäßen Blutbehandlungsvorrichtung 1000 erfolgenden Behandlungsverlauf einer ersten Ausführungsform in einem Diagramm, in welchem die durchgezogene Linie eine tatsächlich anliegende Ultrafiltrationsrate UF_rate einer mittels der Blutbehandlungsvorrichtung 1000 ablaufenden Dialysebehandlung über der Behandlungsdauer t_dialyse dargestellt ist.

Die Ultrafiltrationsbehandlung beginnt zu einem Zeitpunkt t0 mit der vom Arzt an der ersten Eingabevorrichtung 4100 eingegebenen Ultrafiltrationsrate UF_A (oder UF_Ausgang) als Ausgangseinstellung. Zu einem Zeitpunkt t1 macht der Patient P, der eine schonendere Behandlung als die gerade ablaufende wünscht, mittels der zweiten Eingabevorrichtung 4200 eine qualitative Eingabe ("schonend", siehe das "-"-Symbol), welche nach Auswerten der Eingabe und Festlegen der Zieleinstellung dazu führt, dass die Ultrafiltration ab diesem Zeitpunkt abweichend von der Ausgangseinstellung, welche im weiteren Zeitverlauf nur mehr als Strichlinie gezeigt ist, mit der Ultrafiltrationsrate UF_Z (oder UF_Ziel) als Zieleinstellung erfolgt.

Da trotz der vom Patienten zum Zeitpunkt t1 vorgenommenen oder veranlassten Änderung in der hier gezeigten, beispielhaften Ausführungsform eine vorgegeben Ultrafiltrationsleistung oder -menge erzielt werden soll, verlängert sich die Dialysesitzung des Patienten über einen ursprünglich als Behandlungsende avisierten Zeitpunkt t2 hinaus bis zum Zeitpunkt t3, zu welchem trotz der Eingabe des Patienten die vorgegebene Ultrafiltrationsleistung erreicht ist.

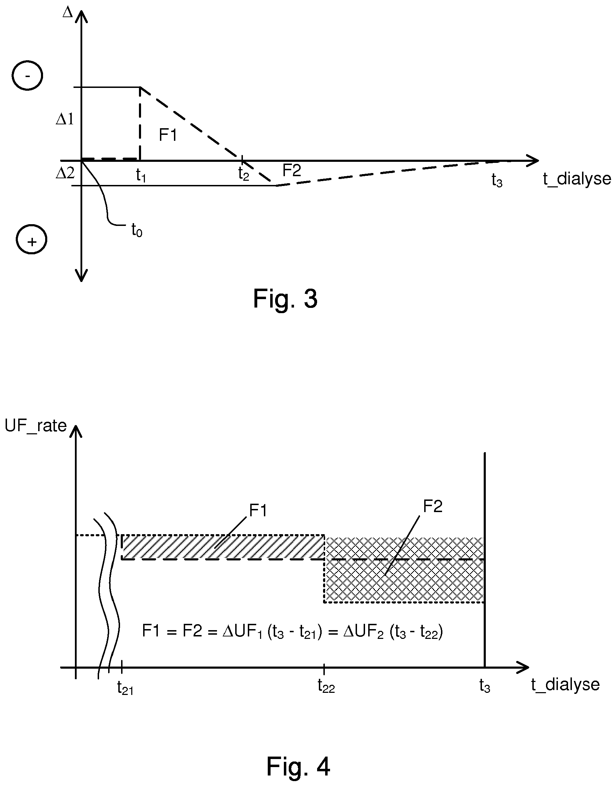

**Fig. 3** zeigt in vereinfachter Darstellung einen exemplarischen, mittels der erfindungsgemäßen Blutbehandlungsvorrichtung erfolgenden Behandlungsverlauf einer zweiten Ausführungsform.

Folgende Erläuterung seien vorweggeschickt: Bei einer Dialysebehandlung können grundsätzlich vier Vorgänge identifiziert werden, welche parallel ablaufen:
a) Entzug von Wasser
b) Entzug von giftigen Substanzen
cl) Korrektur von Elektrolyten (Anpassen von Natrium, Kalium, Magnesium und Calcium)
c2) Korrektur des ph-Wertes durch Zugabe von Bikarbonat

Die Entzugsvorgänge a) und b) führen zu Ungleichgewichten im Körper, die der Patient ggf. spürt und die in der Folge zu den bekannten Symptomen des Unwohlseins führen können.

Jedoch treten Ungleichgewichte während der Dialyse zeitlich nicht konstant auf. Vielmehr kann man eine Dialysebehandlung grob vereinfacht in zwei wesentliche Phasen 1 und 2 unterteilen: In Phase 1 wird der Patient durch den Entzug von osmotisch wirksamen Substanzen belastet ("Desequilibrium Syndrom"). In Phase 2 wird der Patient durch den Entzug von Wasser belastet (ggf. schwere Hypotonie).

In Phase 1 ist der Konzentrationsgradient zwischen Blut und Dialysierflüssigkeit im Blutfilter 3000 kennzeichnend für die Eliminationsgeschwindigkeit von osmotischen Substanzen. Dieser ist zu Beginn der Dialyse bei t0 am höchsten und nimmt dann mit einer e-Funktion mit der Zeit ab. Als Folge des raschen Entzugs von Wassers in Phase 1 entsteht eine Konzentrationsdifferenz für osmotisch wirksame Stoffe zwischen dem Blutkreislauf einerseits und umgebenden Gewebszellen andererseits. Das führt z. B. zum Rückfluss von Plasmawasser in die Zellen des Gehirns und damit dort zu einer Druckerhöhung, die sich durch Kopfschmerzen bemerkbar machen kann. Ähnliche Mechanismen führen zu Herzrhythmusstörungen durch schnellen Abfall von Kalium. Der Patient kann sich in dieser Phase Linderung verschaffen, wenn er den Abtransport der osmotisch wirksamen Stoffe bremst. Dazu kann er mittels der zweiten Eingabevorrichtung 4200, erfindungsgemäß beispielsweise als Regler, Schieber, Kippschalter oder Schalter ausgestaltet, eine als "schonend" beschriebene Eingabe machen. In der Folge reduziert die Blutbehandlungsvorrichtung 1000 die Diffusion von Substanzen aus dem Blut. Hierzu kann eine der folgenden Maßnahmen - oder eine beliebige Kombination hiervon - einleiten:
- Reduzieren oder Stoppen des Flusses der Dialysierflüssigkeit
- Reduzieren des Blutflusses durch den Blutfilter
- Reduzieren der Flussrate vorgenommener Infusionen
- Erhöhen der Ausgangskonzentration der osmotisch wirksamen Substanzen in der Dialysierflüssigkeit
- Reduzieren der Bikarbonat und/oder Natrium-Konzentration in der Dialysierflüssigkeit

Fig. 3 zeigt die Wirkung einer Eingabe des Patienten in einer Darstellung der Abweichung Δ über der Dialysedauer t_dialyse.

Im Beispiel der Fig. 3 hat der Patient zum Zeitpunkt t1 eine "schonendere" Behandlung gewünscht und eine entsprechende Eingabe gemacht. Diese Eingabe wird mittels der Steuervorrichtung 4000 in eine Zieleinstellung "übersetzt", mittels welcher ab dem Zeitpunkt t1 behandelt wird.

Die Eingabe kann wahlweise qualitativ als Impuls oder als Impuls mit quantitativer Intensitätsinformation erfolgen. Die Eingabe wird als Impuls Δ1 für einen oder mehrere der zuvor genannten Parameter gleichzeitig übersetzt. Wenn die Eingabe einen Impuls darstellt, ist die Größe Δ1 ein fester Wert, andernfalls wurde eine festgelegte Übersetzung (in der Software hinterlegt) zwischen der Eingabe des Patienten P und der absoluten Größe von Δ1 vorbestimmt.

Die Tatsache, dass die Belastung des Körpers durch die osmotischen Verschiebungen vom Dialysestart t0 an degressiv abnimmt, führt zu folgenden weiteren Überlegungen:
a) Sowohl der feste Wert für Δ1, als auch die Übersetzung einer variablen Eingabe kann vom Zeitpunkt t1, also von der bislang verstrichenen Behandlungszeit, abhängig gemacht werden. Δ1 kann erfindungsgemäß in bestimmten Ausführungsformen somit je nach der Zeit, die seit Behandlungsbeginn bei t0 verstrichen ist, unter Rückgriff auf in der Steuervorrichtung 4000 hinterlegter Routinen, Algorithmen oder dergleichen verringert oder verstärkt werden. Damit kann erreicht werden, dass die gespürte Auswirkung des Eingriffs des Patienten im Zeitraum der Phase 1 weitestgehend dem gewünschten Nutzen entspricht.
b) Der korrigierende Eingriff wird in der hier diskutierten Ausführungsform bis zum Ende von Phase 1 (t2, Phase 1 dauert von t0 bis t2; Phase 2 beginnt ab t2) von der Steuervorrichtung 4000 automatisch wieder zurückgefahren. Dies geschieht idealerweise progressiv, um der degressiven Entwicklung der Konzentrationsunterschiede zwischen Blut und Dialysierflüssigkeit entgegenzuwirken. Als Resultat ergibt sich dann ein linearer Konzentrationsverlauf der osmotisch wirksamen Stoffe im Blut, wie in Fig. 3 gezeigt.

Der Eingriff in die Flussraten der eingesetzten Pumpen führt zu einer Minderung der Reinigungsleistung (bezogen auf das Blut) mittels der aktuellen Behandlungssitzung. Die Minderleistung entspricht in Fig. 3 beispielhaft der mit F1 bezeichneten Dreiecksfläche.

Da zum Zeitpunkt t2 die Konzentration von Schadstoffen und Elektrolyten im Blut weiter vom zugehörigen Sollwert (dargestellt durch die Zeitachse in Fig. 3) abweicht als dies ohne den Eingriff des Patienten der Fall wäre, ergibt sich für die Restzeit bis zum Ende der Dialyse zum Zeitpunkt t3 automatisch ein gewisser Aufholeffekt. Letzter reicht allerdings nicht aus, um am Ende der Dialysebehandlung dieselbe Reinigungsleistung zu erzielen, als dies ohne den zu t1 erfolgten Eingriff erzielt worden wäre. Aus diesem Grund könnte optional eine Gegenkorrektur der Parameter um Δ2 in Phase 2 erfolgen, um diese Differenz ganz oder teilweise zu kompensieren. Die Wirkung der Gegenkorrektur ist in Fig. 3 als die mit F2 bezeichnete Dreiecksfläche zu verstehen.

**Fig. 4** zeigt in vereinfachter Darstellung einen exemplarischen, mittels der erfindungsgemäßen Blutbehandlungsvorrichtung erfolgenden Behandlungsverlauf in einer dritten Ausführungsform.

In Phase 1, siehe die Ausführungen zu Fig. 3, oder generell in der initialen Phase der Dialysebehandlung, bereitet die Elimination von Wasser dem Körper des Patienten in der Regel wenig Probleme. Das überschüssige Wasser (die Überwässerung) befindet sich sowohl im Gewebe, als auch in den Zellen und im Blutgefäßsystem. Die Blutgefäße sind dadurch leicht expandiert. Der Entzug von Wasser aus dem Gefäßsystem führt zunächst zu einer Entspannung, die der Patient eher als positiv empfindet.

Mit zunehmendem Entzug von Plasmawasser aus dem Gefäßsystem sinkt der Blutdruck allerdings weiter ab. Aus dem die Gefäße umgebenden Gewebe fließt zwar Wasser in die Gefäßlumina nach, dennoch versucht der Körper durch zunehmende Pulsfrequenz und durch eine Konstriktion der peripheren Gefäße, den Blutdruck aufrecht zu erhalten. Das führt zu einer spürbaren Belastung des Patienten. Unter Umständen ist der Körper überdies nicht in der Lage, durch die vorstehend genannten Mechanismen zu kompensieren, und der Patient kann bedenklich hypoton werden.

Der Patient P kann nun gegenregeln, indem er (beispielsweise indirekt mittels des "schonend/effizient"-Reglers als zweite Eingabevorrichtung 4200) die Ultrafiltrationsrate UF_rate, also die Entzugsrate für Flüssigkeit, reduziert. Dadurch wird das Blutvolumen (der Füllgrad des Gefäßsystems) sich erhöhen und der Blutdruck stabilisiert.

Fig. 4 zeigt die Blutbehandlung in der oben als Phase 2 beschriebenen Phase. Wie in Phase 1 kann der Eingriff des Patienten P, veranlasst durch Eingabe in die zweite Eingabevorrichtung 4200, welcher in Fig. 4 zum Zeitpunkt t21 erfolgt, qualitativ als Impuls oder quantitativ als Intensitätsinformation erfolgen.

Der Eingriff in die Ultrafiltrationsrate-Rate UF-rate kann von der Software der Steuervorrichtung 4000 gemäß vorzugsweise wenigstens drei Optionen a), b) oder c) interpretiert werden:
a) Konstant: d. h., die Ultrafiltrationsrate-Rate UF_rate bleibt bis zum Ende der Dialyse begrenzt. Als Konsequenz ergibt sich eine Abweichung vom verschriebenen Trockengewicht nach Dialyse unabhängig vom Zeitpunkt (kann beispielsweise t21 oder beispielsweise t22 sein, siehe Fig. 4), an dem der Patient eingegriffen hat. Fig. 4 zeigt diese Option mit jeweils zwei unterschiedlichen Verläufen, einmal dargestellt mittels Punktlinie, einmal mittels Strichlinie.
b) Abschmelzend: d. h, die Ultrafiltrationsrate-Rate UF-rate wird zurückgenommen und bis zum Ende der Dialyse, d. h. bis zum Zeitpunkt t3, wieder auf den Normalwert hochgefahren.
c) Kompensierend: d. h., die Ultrafiltrationsrate-Rate UF_rate wird zurückgenommen und die Restzeit der Dialyse so aufgeteilt, dass zwar aktuell weniger Flüssigkeit entzogen wird, anschließend hingegen mehr Flüssigkeit entzogen wird, um das vom Arzt verschriebene Trockengewicht zu erreichen.

Sowohl die Auswahl der Optionen a) bis c), als auch die maximal zulässige Höhe der Gewichtsabweichung am Ende der Dialyse, und damit die zulässige Korrektur der Ultrafiltrationsrate-Rate UF_rate zum Zeitpunkt des Eingriffs durch den Patienten wird für jeden Patienten vom Arzt individuell bestimmt und in der Software hinterlegt.

Mit Bezug auf die Fig. 3 und 4 wurde nur die Auswirkung von "schonender" beschrieben, die die Belastung des Patienten reduziert. Insbesondere bei der Ultrafiltrationsrate-Rate UF_rate ist aber auch "effizienter" denkbar. Hier würde die Ultrafiltrationsrate-Rate UF_rate dann um einen Betrag angehoben und das verschriebene Trockengewicht am Ende der Dialyse unterschritten. Sinngemäß gelten die gleichen Mechanismen, wie beschrieben. Bei dieser Variante versteht der Patient P "effizient" dabei als "mehr trinken dürfen".

Als technische Auswirkung dieser Einstellung wird die Ultrafiltrationsrate-Rate UF_rate beispielsweise bereits in Phase 1 nach oben korrigiert, sofern der Patient P es zu dieser Zeit durch "effizienter" auslöst.

Die obigen Ausführungen betreffen eine gedankliche Aufteilung der Behandlungsdauer in zwei Phasen. Es ist aber auch denkbar mehr Phasen einzurichten. Z. B. könnte es eine Phase 1' zwischen Phase 1 und Phase 2 geben, in der die Bedienung der zweiten Eingabevorrichtung 4200, etwa durch Einstellen von "schonender" oder "effizienter", keine Auswirkung hat, eine Zieleinstellung also nicht erstellt wird. Solche weiteren Phasen, ihr Beginn und Ende und dergleichen kann im *setup* der Blutbehandlungsvorrichtung 1000 und/oder in der Steuervorrichtung 4000 festgelegt sein.

Betätigt der Patient P die zweite Eingabevorrichtung 4200, beispielsweise innerhalb eines vorbestimmten Zeitfensters, mehrfach, so kann die Steuerung jede Auslösung in eine Gewichtsdifferenz bei Ende der Dialyse umrechnen, welche durch einen vorgegebenen Grenzwert limitiert werden kann.

### Bezugszeichenliste

- 1: arterieller Leitungsabschnitt
- 2: Dialysatleitung
- 3: venöser Leitungsabschnitt
- 4: Dialysierflüssigkeitsleitung

- 1000: Blutbehandlungsvorrichtung
- 2000: extrakorporaler Blutkreislauf
- 3000: Blutbehandlungseinrichtung, Blutfilter oder Dialysator
- 4000: Steuer- oder Regelvorrichtung
- 4100: erste Eingabevorrichtung
- 4200: zweite Eingabevorrichtung
- 4300: Anzeigevorrichtung

- P: Patient
- t_dialyse: Behandlungsdauer einer Dialysebehandlung

- t0: Beginn der Behandlung
- t1: Zeitpunkt während der Behandlung; Eingriff des Patienten
- t2: Zeitpunkt während der Behandlung
- t3: Ende der Behandlung

- UF_A: Ultrafiltrationsrate als Ausgangseinstellung
- UF_rate: Ultrafiltrationsrate
- UF_Z: Ultrafiltrationsrate als Zieleinstellung

## Patentansprüche

1. Steuer- und/oder Regelungsvorrichtung (4000), welche mit einer ersten, insbesondere von einem Arzt betätigbaren Eingabevorrichtung (4100) und mit einer zweiten, insbesondere von einem Patienten (P) betätigbaren Eingabevorrichtung (4200) jeweils in Signalverbindung verbunden ist, wobei die Steuer- oder Regelungsvorrichtung (4000) programmiert ist, um in Signalverbindung mit einer Blutbehandlungsvorrichtung (1000), welche wiederum mit einem Blutfilter (3000) verbunden ist, eine Blutbehandlung des Patienten (P) unter Ausführen folgender Schritte zu steuern und/oder zu regeln:
- Steuern und/oder Regeln der Blutbehandlungsvorrichtung (1000) zur Blutbehandlung des Patienten (P) mit einer ersten, als Ausgangseinstellung bezeichneten, mittels der ersten Eingabevorrichtung (4100) eingestellten Einstellung für wenigstens einen Behandlungsparameter;
- Überprüfen, ob mittels der zweiten Eingabevorrichtung (4200) eine Eingabe erfolgt ist;
- Auswerten der mittels der zweiten Eingabevorrichtung (4200) erfolgten Eingabe und/oder Festlegen einer zweiten, als Zieleinstellung bezeichneten Einstellung für den wenigstens einen Behandlungsparameter basierend auf der mittels der zweiten Eingabevorrichtung (4200) erfolgten Eingabe; und
- Steuern oder Regeln der Blutbehandlungsvorrichtung (1000) zur Blutbehandlung des Patienten (P) mit der zweiten Einstellung und/oder Steuern oder Regeln der Blutbehandlungsvorrichtung (1000) unter Anpassen oder Ändern der Ausgangseinstellung entsprechend der Eingabe oder der Zieleinstellung;
wobei die zweite Eingabevorrichtung (4200) qualitative Eingaben ermöglicht, und wobei die Steuer- oder Regelungsvorrichtung (4000) programmiert ist, um die mittels der zweiten Eingabevorrichtung (4200) erfolgte, qualitative Eingabe nach vorbestimmten Routinen oder Regeln in Zieleinstellungen zu transformieren.

2. Steuer- und/oder Regelungsvorrichtung (4000) nach Anspruch 1, programmiert, um eine Eingabe mittels der zweiten Eingabevorrichtung (4200), das Festlegen der Zieleinstellung und/oder das Anpassen oder Ändern der Ausgangseinstellung des wenigstens einen Behandlungsparameters nur innerhalb eines zuvor festgelegten Rahmens oder Bereichs oder zuvor festgelegter Grenzen zuzulassen.

3. Steuer- und/oder Regelungsvorrichtung (4000) nach einem der vorangegangenen Ansprüche, wobei der Behandlungsparameter ausgewählt ist aus der Gruppe, bestehend aus: Fluss der Dialysierflüssigkeit, Blutfluss durch den Blutfilter; Zufluss einer Infusionslösung, Bereitstellen oder Konzentration von osmostisch wirksamen Substanzen in der Dialysierflüssigkeit, Konzentration von Bikarbonat, Natrium, Kalium, Magnesium und/oder Kalzium jeweils in der Dialysierflüssigkeit, Filtrationsrate, Ultrafiltrationsrate , Zeitdauer der Blutbehandlung und Temperatur der Dialysierflüssigkeit.

4. Steuer- und/oder Regelungsvorrichtung (4000) nach einem der vorangegangenen Ansprüche, derart ausgestaltet, dass man bei der Eingabe mittels der zweiten Eingabevorrichtung (4200) zwischen einer ersten und einer zweiten Eingabeoption auswählen kann, welche vorzugsweise jeweils ein zueinander entgegen gesetztes Anpassen oder Ändern der Ausgangseinstellung des Behandlungsparameters bewirken.

5. Steuer- und/oder Regelungsvorrichtung (4000) nach einem der vorangegangenen Ansprüche, programmiert, um das Anpassen oder Ändern der Ausgangseinstellung des Behandlungsparameters und/oder das Festlegen der Zieleinstellung in Anhängigkeit von der seit Beginn der Blutbehandlung verstrichenen Zeit (t1) zu bewirken.

6. Steuer- und/oder Regelungsvorrichtung (4000) nach einem der vorangegangenen Ansprüche, programmiert, um ein zu einem ersten Zeitpunkt (t1) oder in einem ersten Zeitraum (t2-t1) während der Blutbehandlung erfolgtes Anpassen oder Ändern der Ausgangseinstellung des Behandlungsparameters mittels der zweiten Eingabevorrichtung (4200) zu einem zweiten Zeitpunkt (t2) oder in einem zweiten Zeitraum (t3-t2) während der Blutbehandlung automatisch mit einer dritten, hier als Korrektureinstellung bezeichneten Einstellung zu steuern oder zu regeln, wobei der zweite Zeitpunkt oder Zeitraum nach dem ersten Zeitpunkt bzw. Zeitraum liegt.

7. Steuer- und/oder Regelungsvorrichtung (4000) nach Anspruch 6, wobei die Korrektureinstellung derart bestimmt wird, dass ein mittels Ausgangseinstellung angestrebtes Ziel der Blutbehandlung erreicht wird.

8. Steuer- und/oder Regelungsvorrichtung (4000) nach Anspruch 7, wobei das angestrebte Ziel ein Hydrierungszustand und/oder ein Entgiftungszustand ist.

9. Steuer- und/oder Regelungsvorrichtung (4000) nach Anspruch 6, wobei die Korrektureinstellung derart bestimmt wird, dass eine Auswirkung des Abweichens von der Ausgangseinstellung zumindest teilweise kompensiert, aufgehoben oder ihr entgegen gewirkt wird.

10. Steuer- und/oder Regelungsvorrichtung (4000) nach einem der vorangegangenen Ansprüche, derart programmiert, dass eine mittels der zweiten Eingabevorrichtung (4200) erfolgte Eingabe und/oder das Festlegen der Zieleinstellung nur erfolgt, wenn die Eingabe während einer vorbestimmten Zeitphase der Blutbehandlung erfolgt.

11. Steuer- und/oder Regelungsvorrichtung (4000) nach einem der vorangegangenen Ansprüche, derart programmiert, dass eine mittels der zweiten Eingabevorrichtung (4200) erfolgte Eingabe mittels Sprache und/oder Geste erfolgen kann.

12. Blutbehandlungsvorrichtung (1000), aufweisend einen extrakorporalen Blutkreislauf (2000), und einen Blutfilter (3000), oder hiermit jeweils verbunden, wobei die Blutbehandlungsvorrichtung (1000) weiter aufweist oder in Signalverbindung verbunden ist mit:
- wenigstens einer Steuer- und/oder Regelungsvorrichtung (4000) nach einem der vorangegangenen Ansprüche.

13. Blutbehandlungsvorrichtung (1000) nach Anspruch 12, ausgestaltet als Hämodialysevorrichtung, Hämofiltrationsvorrichtung oder Hämodiafiltrationsvorrichtung.

## Claims

1. A control and/or regulating apparatus (4000), which is respectively connected via signal link to a first input apparatus (4100), which is in particular operable by a doctor, and to a second input apparatus (4200), which is in particular operable by a patient (P), wherein the control or regulating apparatus (4000) is programmed to control and/or to regulate in signal link with a blood treatment apparatus (1000), which in turn is connected to a blood filter (3000), a blood treatment of the patient (P), by performing the following steps:
- controlling and/or regulating the blood treatment apparatus (1000) for the blood treatment of the patient (P), with a first setting, referred to as original setting, by using the first input apparatus (4100) for at least one treatment parameter;
- checking, whether an input has occurred using the second input apparatus (4200);
- evaluating the input entered using the second input apparatus (4200) and/or specifying a second setting, referred to as target setting, for the at least one treatment parameter based on the input entered using the second input apparatus (4200); and
- controlling or regulating the blood treatment apparatus (1000) for the blood treatment of the patient (P) with the second setting and/or controlling or regulating the blood treatment apparatus (1000) by adjusting or modifying the original setting according to the input or the target setting;
wherein the second input apparatus (4200) enables qualitative inputs, and wherein the control or regulating apparatus (4000) is programmed to transform the qualitative input entered using the second input apparatus (4200) into target settings according to predetermined routines or rules.

2. The control and/or regulating apparatus (4000) according to claim 1, programmed to allow an input using the second input apparatus (4200), the specifying of the target setting and/or the adjusting or modifying of the original setting of the at least one treatment parameter only within a predefined frame or area or within predefined limits.

3. The control and/or regulating apparatus (4000) according to anyone of the preceding claims, wherein the treatment parameter is chosen from the group consisting of:
flow of dialysis liquid, blood flow through the blood filter, inflow of an infusion solution, provision or concentration of osmotically active substances in the dialysis liquid, concentration of bicarbonate, sodium, potassium, magnesium and/or calcium, respectively in the dialysis liquid, filtration rate, ultrafiltration rate,
duration of blood treatment and temperature of the dialysis liquid.

4. The control and/or regulating apparatus (4000) according to anyone of the preceding claims, designed such that, when entering input using the second input apparatus (4200), a first or a second input option can be chosen, which, preferably each, causes a mutually opposite adaptation or modification of the original setting of the treatment parameter.

5. The control and/or regulating apparatus (4000) according to anyone of the preceding claims, programmed to adapt or modify the original setting of the treatment parameter and/or to specify the target setting depending on the time point (t1) elapsed since the beginning of the blood treatment.

6. The control and/or regulating apparatus (4000) according to anyone of the preceding claims, programmed to control or regulate an adjusting or modifying of the original setting of the treatment parameter, occurred at a first time point (t1) or in a first time period (t2-t1) during the blood treatment, using the second input apparatus (4200) at a second time point (t2) or in a second time period (t3-t2) during the blood treatment, automatically with a third setting, described herein as correction setting, wherein the second time point or time period is after the first time point or time period, respectively.

7. The control and/or regulating apparatus (4000) according to claim 6, wherein the correction setting is determined such that a target of the blood treatment aimed at by the original setting is achieved.

8. The control and/or regulating apparatus (4000) according to claim 7, wherein the aimed target is a hydration state and/or a detoxification state.

9. The control and/or regulating apparatus (4000) according to claim 6, wherein the correction setting is determined such that an effect of the deviation from the original setting is, at least partially, compensated, cancelled or counteracted.

10. The control and/or regulating apparatus (4000) according to anyone of the preceding claims, programmed such that an input entered using the second input apparatus (4200) and/or the specifying of the target setting only occur(s) if the input is made during a predetermined time period of the blood treatment.

11. The control and/or regulating apparatus (4000) according to anyone of the preceding claims, programmed such that an input entered using the second input apparatus (4200) can be made using speech and/or gestures.

12. A blood treatment apparatus (1000) comprising an extracorporeal blood circuit (2000), and a blood filter (3000), or respectively connected thereto, wherein the blood treatment apparatus (1000) further comprises or is connected via signal link to:
- at least one control and/or regulating apparatus (4000) according to anyone of the preceding claims.

13. The blood treatment apparatus (1000) according to claim 12, designed as a hemodialysis apparatus, hemofiltration apparatus or hemodiafiltration apparatus.

## Revendications

1. Un appareil de commande et/ou de régulation (4000), qui est relié par signal respectivement à un premier appareil d'entrée (4100), actionnable en particulier par un médecin, ainsi qu'à un second appareil d'entrée (4200), actionnable en particulier par un patient (P), où l'appareil de commande ou de régulation (4000) est programmé pour commander et/ou réguler un traitement du sang d'un patient (P) par liaison de signal avec un appareil de traitement du sang (1000), lui-même relié à un filtre sanguin (3000), en effectuant les étapes suivantes:
- commander et/ou réguler, l'appareil de traitement du sang (1000) destiné au traitement du sang du patient (P), au moyen d'un premier réglage, appelé réglage original, et ajusté au moyen du premier appareil d'entrée (4100) pour au moins un paramètre de traitement;
- vérifier si une entrée a été effectuée au moyen du second appareil d'entrée (4200);
- évaluer l'entrée effectuée au moyen du second appareil d'entrée (4200) et/ou spécifier un second réglage, appelé réglage cible, pour au moins un paramètre de traitement en fonction de l'entrée effectuée au moyen du second appareil d'entrée (4200); et
- commander ou réguler l'appareil de traitement du sang (1000) destiné au traitement du sang d'un patient (P) avec le second réglage et/ou commander ou réguler l'appareil de traitement du sang (1000) en ajustant ou en modifiant le réglage original correspondant à l'entrée ou au réglage cible;
où le second appareil d'entrée (4200) permet des entrées qualitatives, et où l'appareil de commande ou de régulation (4000) est programmé pour transformer l'entrée qualitative effectuée au moyen du second appareil d'entrée (4200) en réglages cibles selon des routines ou des règles prédéterminées.

2. L'appareil de commande et/ou de régulation (4000) selon la première revendication, programmé pour permettre une entrée au moyen du second appareil d'entrée (4200), la spécification du réglage cible et/ou l'ajustement ou la modification du réglage original d'au moins un paramètre de traitement uniquement dans un cadre ou une zone prédéfini(e) ou dans des limites prédéfinies.

3. L'appareil de commande et/ou de régulation (4000) selon l'une quelconque des revendications précédentes, où le paramètre de traitement est choisi parmi le groupe constitué de:
un flux du liquide de dialyse, un flux de sang au travers du filtre sanguin, un afflux d'une solution de perfusion, la fourniture ou la concentration de substances osmotiquement actives dans le liquide de dialyse, une concentration de bicarbonate, de sodium, de potassium, de magnésium et/ou de calcium, respectivement dans le liquide de dialyse, un taux de filtration, un taux d'ultrafiltration, une durée du traitement du sang et une température du liquide de dialyse.

4. L'appareil de commande et/ou de régulation (4000) selon l'une quelconque des revendications précédentes, conçu de telle sorte que, lors de l'entrée au moyen du second appareil d'entrée (4200), une première ou une seconde option d'entrée peut être choisie, qui, de préférence chacune d'elle, provoque une adaptation ou une modification mutuellement opposée du réglage original du paramètre de traitement.

5. L'appareil de commande et/ou de régulation (4000) selon l'une quelconque des revendications précédentes, programmé pour provoquer une adaptation ou une modification du réglage original du paramètre de traitement et/ou la spécification du réglage cible en fonction du temps écoulé (t1) depuis le début du traitement du sang.

6. L'appareil de commande et/ou de régulation (4000) selon l'une quelconque des revendications précédentes, programmé pour, à un second instant (t2) ou durant une seconde période de temps (t3-t2) du traitement du sang, automatiquement commander ou réguler une adaptation ou une modification du réglage original du paramètre de traitement ayant été effectuée au moyen du second appareil d'entrée (4200) à un premier instant (t1) ou durant une première période de temps (t2-t1) du traitement du sang,
avec un troisième réglage, désigné comme réglage de correction, le second instant ou la seconde période de temps étant postérieure au premier instant ou à la première période de temps.

7. L'appareil de commande et/ou de régulation (4000) selon la revendication 6, où le réglage de correction est déterminé de manière à ce qu'un objectif du traitement du sang visé au moyen du réglage original soit atteint.

8. L'appareil de commande et/ou de régulation (4000) selon la revendication 7, où l'objectif visé est un état d'hydrogénation et/ou un état de détoxification.

9. L'appareil de commande et/ou de régulation (4000) selon la revendication 6, où le réglage de correction est déterminé de manière à ce qu'un effet de l'écart par rapport au réglage original soit au moins partiellement compensé, annulé ou contrebalancé.

10. L'appareil de commande et/ou de régulation (4000) selon l'une quelconque des revendications précédentes, programmé de manière à ce que l'entrée effectuée au moyen du second appareil d'entrée (4200) et/ou la spécification du réglage cible n'ait(ent) lieu que si l'entrée est effectuée pendant une période de temps prédéterminée du traitement du sang.

11. L'appareil de commande et/ou de régulation (4000) selon l'une quelconque des revendications précédentes, programmé de manière à ce que l'entrée effectuée au moyen du second appareil d'entrée (4200) puisse avoir lieu au moyen de la parole et/ou de gestes.

12. Un appareil de traitement du sang (1000) comprenant un circuit sanguin extracorporel (2000), ainsi qu'un filtre sanguin (3000), ou étant respectivement relié à ceux-ci, où l'appareil de traitement du sang (1000) comprend en outre ou est en liaison de signal avec:
- au moins un appareil de commande et/ou de régulation (4000), selon l'une quelconque des revendications précédentes.

13. L'appareil de traitement du sang (1000) selon la revendication 12, conçu comme appareil d'hémodialyse, appareil d'hémofiltration ou appareil d'hémodiafiltration.
